# EUROPÄISCHE PATENTSCHRIFT

(11) **EP 0 428 127 B1**
(45) Veröffentlichungstag und Bekanntmachung des Hinweises auf die Patenterteilung: **03.08.1994**
(21) Anmeldenummer: 90121682.0
(22) Anmeldetag: 13.11.1990
(51) Int. Cl.: A61F 2/36

(54) **Zementierbare Endoprothese**
Endoprothesis to be inplanted with cement
Endoprothèse à implanter avec ciment

(30) Priorität: 14.11.1989 DE 3937786; 12.04.1990 DE 4011887
(43) Veröffentlichungstag der Anmeldung: 22.05.1991
(73) Patentinhaber: Jansson, V. Dipl.-Ing. Dr. Med ., D-82205 Gilching (DE)
(72) Erfinder: Jansson, V. Dipl.-Ing. Dr. Med ., D-82205 Gilching (DE)
(74) Vertreter: Hoeger, Stellrecht & Partner

(56) Entgegenhaltungen:
- EP-A- 0 315 283
- DE-A- 2 310 113
- DE-A- 3 704 089
- US-A- 4 274 163
- US-A- 4 888 022
- US-A- 4 888 024

## Beschreibung

Die Erfindung betrifft eine zementierbare Endoprothese mit den im Oberbegriff des Patentanspruches 1 angegebenen Merkmalen.

Bei der Implantation von Endoprothesen wurde der in der Zahnheilkunde bekannte Knochenzement von CHARNLEY 1960 erstmals verwendet. Ziel der zementierten Endoprothese ist es, durch den Zement einen festen Verbund zwischen Knochen und Prothese herzustellen. Seitdem wurden verschiedene Zementiertechniken zum optimalen Einbringen des Knochenzementes entwickelt.

Eines der Probleme beim Zementieren ist das möglichst hohlraumfreie Einbringen des Zementes zwischen Prothese und Knochen. Ein weiteres Problem besteht darin, daß es beim Einbringen des Zementes zu Vermischungen des Zementes mit Blut kommt, dies führt nach der Aushärtung zu Einschlüssen von Blut in den Zement, wodurch die Haltbarkeit des Zementes stark vermindert wird. Insbesondere scheinen parallel zu Prothese und Knochen verlaufende Blutschlieren die Festigkeit der einzementierten Prothese stark zu vermindern.

Bei den herkömmlichen Zementiertechniken versucht man, durch Einspritzen des Zementes entweder von proximal oder mit Spritzenverlängerung retrograd von distal eine möglichst vollständige und von Blutbeimischungen freie Zementfüllung zu erzielen. Auch ein Einsaugen des Zementes in die Markraumhöhle bei gleichzeitiger Entlüftung der Markraumhöhle wird praktiziert. Bei diesen Techniken wird dann nach Einbringen des Zementes die Prothese in den zementgefüllten Markraum geschoben. Das Weiterlaufen des Knochenzementes nach distal versucht man durch "Markraumstopper" zu verhindern, die aus resorbierbarem oder nicht resorbierbarem Material oder auch zum Beispiel aus Knochen (Spongiosa) bestehen können. Diese Markraumstopper haben in der Regel keine Verbindung mit der Prothese und funktionieren nach dem Prinzip des Stopfens.

Nachteilig bei all diesen Zementiertechniken ist, daß die vollständige Zementfüllung oft nicht erzielt und der Einschluß von Blut oft nur schlecht verhindert werden kann. Außerdem kommt es oft beim Aushärten durch kleine Bewegungen an der Prothese zu einer Verschlechterung des Formschlusses, da der Zement nur schwer von oben nachgedrückt werden kann.

Es ist bekannt, die Verankerung der Prothese im Knochen dadurch zu verbessern, daß die Prothese mit einer Axialbohrung versehen wird, von der eine Vielzahl von Bohrungen ausgehen. Durch Einpressen des Knochenzements in die Axialbohrung gelangt dieser über die Vielzahl von Bohrungen an unterschiedlichen Stellen der Prothese in den Knochenzwischenraum zwischen Prothese und Knochen, so daß dadurch eine verbesserte und gleichmäßigere Füllung des Zwischenraumes erzielt wird (DE-A-2 310 113). Allerdings bleibt weiterhin das Problem bestehen, daß in den Knochenzement Blut und andere Körperflüssigkeiten eingebettet werden, die die Festigkeit des ausgehärteten Knochenzementes verringern.

Aus der US-A-4 274 163 ist eine Endoprothese bekannt, bei welcher der Zwischenraum zwischen Prothesenschaft und Knochen über eine Entlüftungsbohrung mit dem Außenraum in Verbindung steht. Zwar ist dadurch eine geringfügige Verbesserung bei der Füllung des Zwischenraumes mit dem Knochenzement möglich, jedoch treten auch hier noch die geschilderten Probleme auf.

Ausgehend von diesem Stand der Technik ist es Aufgabe der vorliegenden Erfindung, ein möglichst gleichmäßiges Eindringen des Knochenzementes im gesamten Zwischenraum zu ermöglichen, insbesondere ohne dabei einen besonders großen Druck des Knochenzementes aufzubringen.

Diese Aufgabe wird bei einer Endoprothese der eingangs beschriebenen Art erfindungsgemäß durch die kennzeichnenden Merkmale des Patentanspruches 1 gelöst. Durch das Vorsehen der Absaugöffnungen kann im Zwischenraum zwischen Prothese und Knochen eingeschlossene Luft und eingeschlossene Körperflüssigkeit, insbesondere Blut, abgesaugt werden, so daß die Gefahr verringert wird, daß diese Substanzen in den Knochenzement eingeschlossen werden. Es ist sogar möglich, daß ein Teil des Knochenzementes mit abgesaugt wird, so daß eine regelrechte Durchspülung des Zwischenraumes zwischen Knochen und Prothese erfolgt, bevor der Aushärtvorgang einsetzt. Dadurch ist auf jeden Fall gewährleistet, daß der Knochenzement in dem Zwischenraum nicht verunreinigt ist.

Ein weiterer Vorteil der Kombination der Austrittsöffnungen mit Absaugöffnungen liegt darin, daß insgesamt niedrigere Zementdrucke verwendet werden können, da jeweils nur kleinere Bereiche der Markraumöffnung von einer Austrittsöffnung aus mit Knochenzement versorgt werden müssen.

Durch das gleichzeitige Absaugen der beim Austritt des Knochenzementes in die Markraumhöhle entstehenden Hohlräume durch die Absaugöffnungen wird außerdem ein Wegdrücken der in den Hohlräumen befindlichen Substanzen (Blut, Markraumfett, Luft) in die Blutbahn verhindert. Dadurch verringert sich die Gefahr intraoperativer Embolien.

Beim Eindrücken des Knochenzementes können sich im Zwischenraum zwischen Knochen und Prothesenschaft abgeschlossene Taschen bilden, in denen Luft und/oder Flüssigkeiten eingeschlossen sind. Diese Taschen können durch die Absaugungen geleert werden, so daß die Taschenbildung insgesamt verhindert wird.

Besonders vorteilhaft ist es dabei, wenn die Absaugöffnungen gegenüber den Austrittsöffnungen versetzt sind, so daß jeweils im Bereich zwischen zwei Austrittsöffnungen eine Absaugung stattfindet. Dadurch wird sichergestellt, daß die durch benachbarte Austrittsöffnungen austretenden Knochenzementmengen sich homogen vereinigen können, ohne daß im Kontaktbereich Blut- oder sonstige Fremdkörpereinschlüsse auftreten.

Es ist weiterhin vorteilhaft, wenn der Innendurchmesser der Austritts- und/oder der Absaugöffnungen längs der Prothese entsprechend an den von jeder Öffnung zu versorgenden Teil des Zwischenraumes zwischen Prothese und umgebenden Knochen angepaßt ist. Die Größe des von jeder Öffnung zu versorgenden Teils des Zwischenraumes hängt einerseits vom Abstand der Austrittsöffnungen beziehungsweise der Absaugöffnungen voneinander ab, andererseits von den Querschnitten des aufgeraspelten Markraumes einerseits und des Prothesenschaftes andererseits. Die lichte Weite des Zwischenraumes kann sich somit über die Länge des Prothesenschaftes ändern, und trotzdem wird die pro Zeiteinheit eingepreßte Knochenzementmenge durch die entsprechende Wahl der Innendurchmesser der genannten Bohrungen genau an das zur Verfügung stehende Volumen des Zwischenraumes angepaßt. Auf diese Weise erfolgt die Füllung des Zwischenraumes mit Knochenzement gleichzeitig und gleichmäßig.

Bei einer besonders bevorzugten Ausführungsform ist vorgesehen, daß die Austritts- und/oder Absaugöffnungen im Bereich der Prothesenoberfläche mit einer Schicht eines verformbaren Materials ausgekleidet sind. Nach dem Aushärten des Knochenzementes erstrecken sich Fortsätze des ausgehärteten Knochenzementes in diese Öffnungen hinein. Durch die Auskleidung der Öffnungen mit einem verformbaren Material ist sichergestellt, daß auch bei einer geringfügigen Bewegung der Prothese gegenüber dem Knochen diese Vorsprünge des ausgehärteten Knochenzementes nicht abbrechen, so daß der durch sie bewirkte Formschluß erhalten bleibt. Andererseits führt diese geringfügige Verschiebbarkeit im Zusammenhang mit unterschiedlichen Innendurchmessern dieser Öffnungen dazu, daß bei einem gegebenenfalls notwendig werdenden Herauslösen der Prothese diese Vorsprünge bei einer Verschiebung der Prothese nicht gleichzeitig abbrechen, sondern entsprechend dem jeweiligen Innendurchmesser der Öffnungen nacheinander. Dies erleichtert das Lösen der Prothese.

Um die Markraumhöhle am distalen Ende abzuschließen, kann vorgesehen sein, daß am distalen Ende der Prothese ein das Eintreten von Knochenzement in die Markraumöffnung des Knochens verhindernder Markraumstopper angeordnet ist, dieser ist vorzugsweise an der Prothese fixiert.

Bei einer bevorzugten Ausführungsform kann der Markraumstopper eine zum distalen Ende der Prothese hin aufspannbare, regenschirmförmig ausgebildete Kappe sein, die vorzugsweise aufgespannt ist und sich zentrierend an die Innenwand des Markraumes anlegt. Beim Eindrücken des Knochenzementes in den Zwischenraum zwischen Prothese und Knochen wird diese Kappe zusätzlich aufgespannt und an die Innenwand des Knochens angelegt, so daß dadurch die Abdichtung verbessert wird.

Bei einer anderen Ausführungsform kann an der Prothese als distaler Markraumstopper ein diese umgebender Ring angeordnet sein. Dieser ist bei einem bevorzugten Ausführungsbeispiel ein Schlauch, in den ein fließfähiges Medium einführbar ist, beispielsweise ein Gas, eine Flüssigkeit oder fließfähiger Knochenzement. Diese Ausgestaltung hat den Vorteil, daß sich der Markraumstopper an jede Querschnittsform der Markraumhöhle abdichtend anlegt.

Bei einer weiteren Ausführungsform kann vorgesehen sein, daß der Markraumstopper als sich an das distale Ende der Prothese anschließender Ballon ausgebildet ist, in den ebenfalls ein fließfähiges Medium einführbar ist.

Der Markraumstopper wird normalerweise konzentrisch zum Prothesenschaft ausgebildet, er kann aber auch exzentrisch zum Prothesenschaft ausgebildet sein, so daß dadurch eine Knochenzementschicht entsteht, die auf verschiedenen Seiten der Prothese unterschiedlich dick ist. Dies kann aus Festigkeitsgründen erwünscht sein, so daß es bei geeigneter Ausbildung der Knochenzementschicht möglich ist, lokale Spannungsspitzen zu vermeiden. Diese Spannungsspitzen sind besonders schädlich, da im Bereich dieser Spannungsspitzen ein bevorzugter Abbau des Knochens auftritt.

Ein ringförmig ausgebildeter Markraumstopper muß nicht unbedingt mit der Prothese verbunden sein, er kann auch durch Form- beziehungsweise Reibschluß zwischen Prothese und Knochen haften.

Günstig ist es, wenn der Membranstopper aus einem resorbierbaren oder einem knöchern um- und einbaubaren Material besteht, beispielsweise aus Tricalciumphosphat, Polylactid, etc. beziehungsweise Hydroxylapatit. In anderen Fällen wird der Membranstopper aus einem weichen Material gefertigt, welches sich zuverlässig auch an unregelmäßige Verläufe des Markraumes anpaßt, beispielsweise aus Polyethylen.

Bei einer bevorzugten Ausführungsform ist vorgesehen, daß die Prothese auf der proximalen Seite des distalen Markraumstoppers eine nach innen zurückspringende Ausnehmung zur Aufnahme von Knochenzement aufweist. Dadurch ergibt sich ein Formschluß zwischen dem Knochenzementmantel und dem distalen Prothesenende und dadurch eine zuverlässige Verankerung des Prothesenschaftes im Markraum.

Der distale Membranstopper kann aus einem porösen Material bestehen, dadurch kann die Entlüftung des Markraumes in distaler Richtung unterstützt werden. Der Membranstopper kann auch kompakt ausgebildet sein und entsprechende Absaugbohrungen aufweisen, die in gleicher Weise der Entlüftung des Markraumes dienen können.

Besonders vorteilhaft ist es, wenn an der Prothese als proximaler Markraumstopper ein diese umgebender, gegebenenfalls angeformter Ring angeordnet ist, dessen Außendurchmesser größer ist als der Innendurchmesser des Markraumes. In Verbindung mit dem distalen und diesem proximalen Membranstopper ist der gesamte Zwischenraum zwischen Prothese und Knochen vollständig abgeschlossen und nur über die Knochenzement-Austrittsöffnungen und die Absaugöffnungen zugänglich. Dies gewährleistet die einwandfreie Entlüftung dieses abgeschlossenen Zwischenraumes und die homogene und einschlußfreie Füllung des Zwischenraumes. Wesentlich ist dabei auch, daß durch den Abschluß des Zwischenraumes auf der distalen und auf der proximalen Seite eine Kompression des Knochenzementes eintritt, die zusammen mit der Absaugung durch die Absaugöffnungen die homogene Füllung des Zwischenraumes begünstigt.

Der Ring kann drehbar beziehungsweise gleitend mit der Prothese verbunden sein. Dadurch kann durch Aufpressen der Prothese auf die Osteotomiefläche ein möglichst vollständiger (in Abhängigkeit von der Osteotomiefläche meist schräger) Aufsitz des Ringes auf der Osteotomiefläche erzielt werden.

Vorzugsweise ist der Ring abnehmbar, insbesondere kann er dazu teilbar ausgebildet sein.

Eine besonders wirksame Abdichtung ergibt sich, wenn der Ring zumindest an seiner Unterseite aus einem weichen Kunststoffmaterial besteht, das sich an die Osteotomiefläche anpaßt, beispielsweise Polyethylen.

Bei einer besonders bevorzugten Ausführungsform ist vorgesehen, daß der Ring sich in distaler Richtung soweit verjüngt, daß er mit seinem distalen Ende in die Markraumöffnung einführbar ist. Dadurch ergibt sich ein Zentriersitz. Einerseits wird der Markraum durch das Einpressen der Prothese proximal abgedichtet und andererseits wird die Prothese an diesem Ende im Markraum in der gewünschten Position zentriert.

Der Ring kann als Konus ausgebildet sein, das heißt gerade verlaufende Flanken aufweisen, die Flanken können aber auch gebogen sein. Dabei ist eine symmetrische Ausgestaltung des Ringes möglich, bei einer speziellen Ausführungsform können die Flanken des Ringes längs des Ringumfanges jedoch auch unterschiedlich verlaufen, so daß beim Einpressen der Prothese diese bezüglich der Mittelachse des Markraumes verlagert wird.

Ein besonderer Vorteil des sich verjungenden, proximalen Markraumstoppers liegt auch darin, daß durch das Einbringen des Markraumstoppers in den Markraum die Wandstärke des Knochenzementmantels in proximaler Richtung abnimmt. Daraus ergibt sich insbesondere bei Verwendung eines weichen Materials für den Markraumstopper eine weiche Spannungseinleitung in den Knochen. Im Gegensatz zum Beispiel zu einem Kragenaufsitz am proximalen Knochenschaftende mit entsprechend harter Spannungseinleitung in den Knochen steigt die Steifigkeit des sich in distaler Richtung verdickenden Knochenzementmantels von proximal nach distal an, so daß die von der Prothese auf den Knochen in diesem Bereich übertragenen Spannungen entsprechend diesem nach distal ansteigenden Steifigkeitsverhalten verteilt in den Knochen eingeleitet werden. Derartige weichere Übergänge können ungünstige Spannungsspitzen im proximalen Knochen vermeiden.

Vorteilhaft ist es, wenn die Ebene des Ringes gegenüber der Prothesenschaftachse geneigt ist. Dadurch ergibt sich eine bessere Anpassung an die in der Regel ebenfalls geneigten Osteotomieflächen. Günstig ist es, wenn der Ring aus einem im Vergleich zur übrigen Prothese weichen Material besteht, insbesondere aus einem Material, das durch Fingerdruck leicht komprimierbar ist. Man erhält auch dann noch eine sichere Zentrierung der Prothese und eine sichere Abdichtung der proximalen aufgeraspelten Markraumhöhle, wenn diese fehlerhaft zu weit aufgeraspelt wurde, da sich das weiche Material des Ringes dem Markraum in gewissen Grenzen anpassen kann.

Die Form des Ringes führt weiterhin dazu, daß die Prothese beim Eindrücken in den Markraum die Spongiosa komprimiert und dadurch einerseits eine bessere proximale Abdichtung erzielt und andererseits ein Verklemmen und damit einen festeren Sitz der Prothese während der Phase des Zementeinspritzens herbeiführt.

Der Ring kann bei einem anderen bevorzugten Ausführungsbeispiel auch aus einem resorbierbaren Material bestehen, beispielsweise aus Tricalciumphosphat.

Die nachfolgende Beschreibung bevorzugter Ausführungsformen der Erfindung dient im Zusammenhang mit der Zeichnung der näheren Erläuterung. Es zeigen:
- Figur 1 :: einen Hüftendoprothesenschaft mit einem Doppelbohrsystem für die Zementapplikationen sowie für die Absaugung in Seitenansicht;
- Figur 2 :: eine Ansicht des Hüftendoprothesenschaftes der Figur 1 von oben;
- Figur 3 :: eine Schnittansicht längs Linie 3-3 in Figur 1;
- Figur 4 :: eine Schnittansicht längs Linie 4-4 in Figur 1;
- Figur 5 :: eine Seitenansicht eines Hüftendoprothesenschaftes ähnlich Figur 1 mit einem am distalen Ende austretenden Einführkanal für den Knochenzement;
- Figur 6 :: eine Ansicht des Prothesenschaftes der Figur 5 von oben;
- Figur 7 :: eine Schnittansicht längs Linie 7-7 in Figur 5;
- Figur 8 :: das distale Ende eines in den Markraum eingeführten Prothesenschaftes mit einem regenschirmförmigen Markraumstopper;
- Figur 9 :: eine Ansicht ähnlich Figur 8 mit einem schlauchförmigen Markraumstopper;
- Figur 10 :: eine Längsschnittansicht eines Femurkopfes mit eingesetztem Prothesenschaft und mit proximalem Markraumstopper;
- Figur 11 :: eine Draufsicht auf die beiden Teile des Markraumstoppers der Figur 10;
- Figur 12 :: eine Ansicht ähnlich Figur 8 mit einem als auffüllbaren Ballon ausgebildeten distalen Markraumstopper;
- Figur 13 :: ein Ausschnitt eines Prothesenschaftes mit sich in Ausflußrichtung erweiternden Austrittsöffnungen für den Knochenzement;
- Figur 14 :: eine Seitenansicht eines Endoprothesenschaftes mit einem sich in distaler Richtung verjungenden proximalen Markraumstopper;
- Figur 15 :: eine Schnittansicht eines Prothesenschaftes längs Linie 15-15 in Figur 14;
- Figur 16 :: eine Ansicht ähnlich Figur 15 mit einem Markraumstopper mit gebogenen Flanken;
- Figur 17 :: eine Ansicht ähnlich Figur 16 mit unsymmetrischen Flanken;
- Figur 18 :: eine Längsschnittansicht durch einen Röhrenknochen mit eingesetztem Prothesenschaft;
- Figur 19 :: eine Ansicht des distalen Endes eines Prothesenschaftes mit einem distalen Markraumstopper;
- Figur 20 :: eine Ansicht ähnlich Figur 19 mit einem anderen Ausführungsbeispiel eines distalen Markraumstoppers und
- Figur 21 :: eine Ansicht ähnlich Figur 19 mit einem anderen Ausführungsbeispiel eines distalen Markraumstoppers.

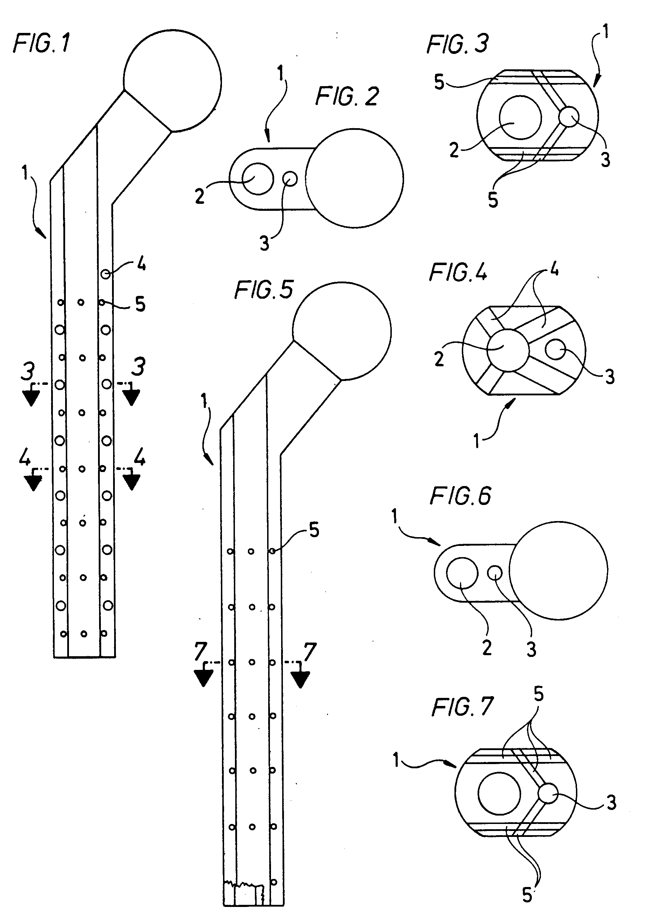

In den Figuren 1 bis 4 wird ein Hüftendoprothesenschaft 1 mit einem voneinander getrennten Doppelbohrsystem dargestellt, welches einen Einführkanal 2 für fließfähigen, aushärtbaren Knochenzement sowie einen parallel dazu durch den Prothesenschaft verlaufenden Absaugkanal 3 umfaßt. Der Einführkanal 2 führt zu einer Vielzahl von aus der Oberfläche des Prothesenschaftes 1 austretenden Austrittsöffnungen 4, die über die gesamte Länge des Prothesenschaftes 1 verteilt sind. Zwischen den Austrittsöffnungen 4 befinden sich in der Außenoberfläche des Prothesenschaftes 1 eine Vielzahl von Absaugöffnungen 5, die zu dem Absaugkanal 3 führen. Zum Einzementieren des Prothesenschaftes wird dieser in den Markraum eingeführt. Durch den Einführkanal 2 wird Knochenzement eingepreßt, der durch die Austrittsöffnungen 4 in den Zwischenraum zwischen Prothesenschaft einerseits und Knochenwand andererseits geleitet wird. Gleichzeitig wird der Absaugkanal 3 an eine in der Zeichnung nicht dargestellte Absaugung angeschlossen, so daß über die Absaugöffnungen 5 Flüssigkeit und Luft aus dem Markraum abgesaugt werden. Insbesondere kann dadurch die unerwünschte Taschenbildung vermieden werden, bei welcher Fremdsubstanzen wie Luft oder Flüssigkeit vom Knochenzement allseits umschlossen werden.

Die Anschlüsse an den Einführkanal 2 und den Absaugkanal 3 befinden sich an der Oberseite des Prothesenschaftes 1.

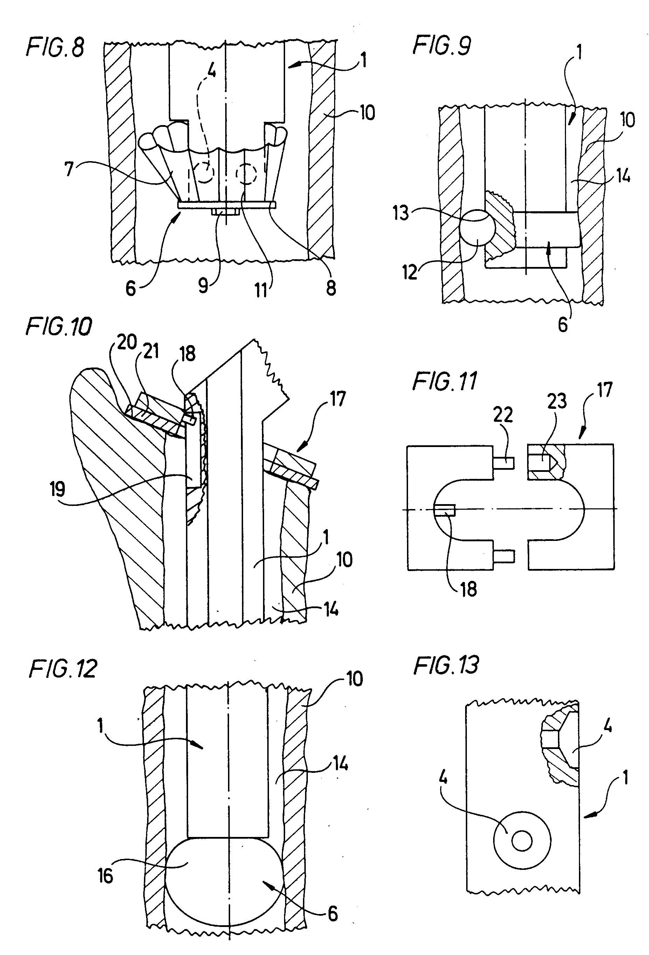

Die Austrittsöffnungen 4 können als normale Bohrungen ausgebildet sein, besonders vorteilhaft ist es jedoch, wenn sie sich nach außen hin erweitern, wie dies in Figur 13 dargestellt ist. Bei diesem Ausführungsbeispiel sind die Austrittsöffnungen 4 trichterförmig aufgebohrt, jedoch sind auch andere geometrische Erweiterungen zur Vergrößerung der Zementaustrittsfläche und zur Verbesserung der Zementverteilung zwischen Prothese und Schaft denkbar. In Figur 13 sind aus Gründen der besseren Übersichtlichkeit die Absaugöffnungen weggelassen.

In den Figuren 5 bis 7 wird ein Hüftendoprothesenschaft 1 dargestellt, der dem den Figuren 1 bis 4 weitgehend entspricht. Gleiche Teile tragen daher dieselben Bezugszeichen. Im Gegensatz zu dem Ausführungsbeispiel der Figuren 1 bis 4 fehlen jedoch vom Einführkanal 2 abzweigende Austrittsöffnungen 4; der Einführkanal 2 mündet am distalen Ende des Prothesenschaftes 1 in den Markraum, so daß der Markraum (in Verbindung mit einem Markraumstopper) von distal nach proximal mit Zement gefüllt wird.

In Figur 8 wird ein distaler Markraumstopper 6 dargestellt, bei dem eine Kunststoffolie 7 mittels einer Scheibe 8 und einer Schraube 9 am distalen Ende des Prothesenschaftes 1 befestigt ist. Durch den aus den Austrittsöffnungen 4 austretenden Zement legt sich die Folie 7 an den Knochen 10 an. In der Folie befestigte Stäbe 11, die sich auf der Scheibe 8 abstützen, verhindern ein Durchschlagen der Kunststoffolie 7. Insgesamt ist die Kunststoffolie 7 mit den Stäben 6 nach Art eines Regenschirmes ausgebildet.

In Figur 9 wird ein distaler Markraumstopper 6 dargestellt, bei dem ein ringförmiger Schlauch 12 in eine Nut 13 am Prothesenende eingelegt ist, der vor oder während des Zementierens durch Luft, Flüssigkeit oder Knochenzement aufgefüllt wird und so den Zwischenraum 14 zwischen Knochen 10 und Prothesenschaft 1 verschließt.

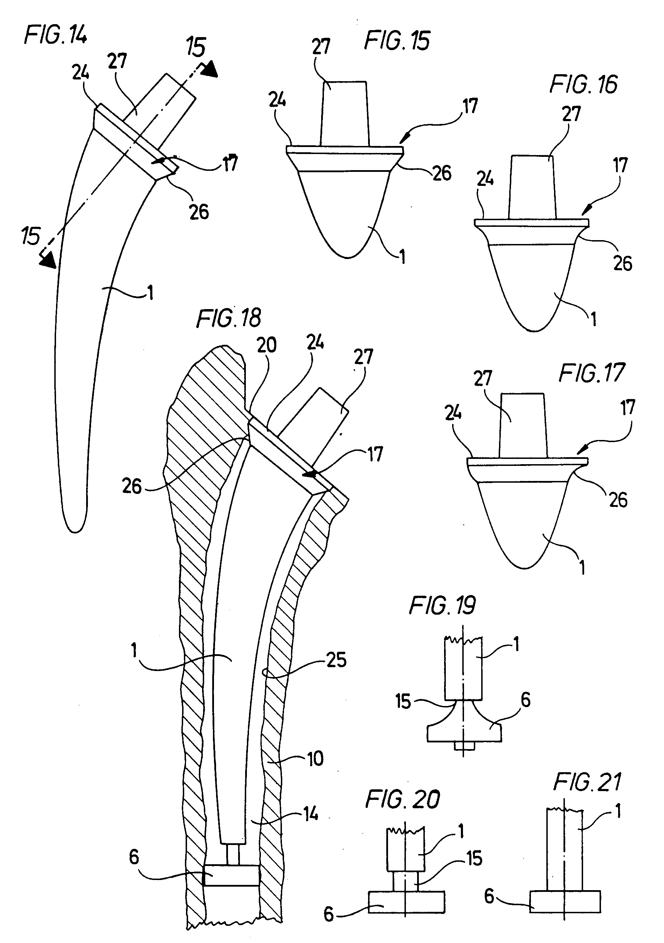

Weitere Ausführungsformen von distalen Markraumstoppern 6 sind den Figuren 19, 20 und 21 zu entnehmen.

Bei den Ausführungsformen der Figuren 19 und 20 befindet sich am proximalen Ende der im wesentlichen scheibenförmig ausgebildeten Markraumstopper 6 eine nach innen rückspringende Ausnehmung 15, die im dargestellten Ausführungsbeispiel an der proximalen Seite stufenförmig ausgebildet ist. Dadurch kann der Knochenzement zwischen dem distalen Ende des Prothesenschaftes 1 und dem proximalen Ende des Markraumstoppers 6 eintreten und einen Formschluß erzeugen.

Im Ausführungsbeispiel der Figur 21 fehlt eine Ausnehmung 15, dort schließt der Markraumstopper 6 unmittelbar an den Prothesenschaft 1 an.

Eine weitere Ausführungsform eines distalen Markraumstoppers 6 ist in Figur 12 dargestellt. Dabei ist ein Ballon 16 am Ende des Prothesenschaftes 1 befestigt, der vor oder während des Zementierens durch Luft, Flüssigkeit oder Knochenzement aufgefüllt wird und so den Zwischenraum 14 zwischen Knochen 10 und Prothesenschaft 1 verschließt.

In Figur 10 wird ein proximaler Markraumstopper 17 dargestellt, der durch eine Nase 18 in eine Nut 19 des Prothesenschaftes 1 eingreift. Der Markraumstopper liegt der Prothese ringförmig an. Durch diese im Rahmen des Spiels zwischen Markraumstopper 17 und Prothesenschaft 1 drehbare und gleitende Lagerung wird ein Aufpressen des Markraumstoppers auf die Osteotomiefläche 20 beim Eindrücken der Prothese erzielt. Zur besseren Abdichtung auf der Osteotomiefläche besteht der untere Teil 21 des Markraumstoppers 17 aus einem weichen Material, beispielsweise aus Polyethylen.

Der Markraumstopper der Figur 10 ist, wie sich aus der Darstellung der Figur 11 ergibt, geteilt ausgeführt und kann durch ineinandergreifende Zapfen 22 und Bohrungen 23 um den Prothesenschaft 1 herum zusammengesteckt werden.

Während der proximale Markraumstopper 17 gemäß der Darstellung der Figuren 10 und 11 im wesentlichen als ebene Platte ausgebildet ist und flächig an der Osteotomiefläche 20 anliegt, ist bei dem in Figur 18 dargestellten Ausführungsbeispiel ein proximaler Markraumstopper 17 vorgesehen, der als am Prothesenschaft 1 festgelegter Ring 24 ausgebildet ist, welcher sich in distaler Richtung soweit verjüngt, daß das distale Ende in den Markraum 25 eintritt, während das proximale Ende entweder bündig mit der Osteotomiefläche 20 abschließt oder über diese hervorsteht. Bei dem in Figur 18 dargestellten Ausführungsbeispiel ist der Ring als Konus ausgebildet, das heißt die Flanken 26 sind eben. Außerdem ist dieser Konus relativ zur Längsachse des Prothesenschaftes 1 zentriert. Eine solche Ausgestaltung ergibt sich auch aus den Darstellungen der Figuren 14 und 15. In den Figuren 16 und 17 sind dagegen abweichende Geometrien dargestellt, bei der Ausführung der Figur 16 sind die Flanken 26 symmetrisch gebogen, bei der Ausführung der Figur 17 sind die Flanken auch gebogen, jedoch unsymmetrisch.

Bei den symmetrischen Ausgestaltungen ergibt sich beim Eindrücken des Prothesenschaftes in den Markraum eine zentrale Positionierung, bei dem Ausführungsbeispiel der Figur 17 dagegen eine exzentrische Justierung, so daß der Knochenzementmantel längs des Umfanges unterschiedlich dick ausgebildet wird.

Der in Figur 18 dargestellte Prothesenschaft 1 weist im übrigen in üblicher Weise an seinem proximalen Ende einen Steckkonus 27 für eine nicht dargestellte Gelenkkugel auf sowie am distalen Ende einen distalen Markraumstopper 6, der beispielsweise in der in Figur 20 dargestellten Weise ausgebildet sein kann.

Die Prothese wird im Markraum 25 sowohl durch den distalen Markraumstopper 6 als auch durch den proximalen Markraumstopper 17 justiert und festgelegt, wobei der Zwischenraum 14 beidseitig abgeschlossen ist. In dieser Position kann die Einfüllung des Zementes bei gleichzeitiger Absaugung erfolgen, die Fixierung des Prothesenschaftes bleibt allein durch die beiden Markraumstopper erhalten, bis der Zement ausgehärtet ist.

Die Ebene des als oberer Markraumstopper wirkenden Ringes 24 kann senkrecht zur Längsachse des Prothesenschaftes angeordnet sein, jedoch ist auch eine Neigung gegenüber dieser Längsachse möglich, so daß die Ebene des Ringes im wesentlichen mit der Osteotomiefläche 20 zusammenfällt.

## Patentansprüche

1. Zementierbare Endoprothese mit einem in einen Knochenhohlraum einsetzbaren Prothesenschaft (1), mit einem Einführkanal (2) für fließfähigen und aushärtbaren Knochenzement, mit mindestens einer aus diesem Einführkanal (2) zur Außenfläche des Prothesenschaftes führenden Austrittsöffnung (4), durch welche Knochenzement in den Zwischenraum zwischen Prothese und umgebenden Knochen eindrückbar ist, und mit einer Verbindung in der Prothese zur Verbindung des Zwischenraumes zwischen Prothese und umgebendem Knochen nach außen bei in den Knochenhohlraum eingesetzter Endoprothese,
dadurch gekennzeichnet, daß die Verbindung in der Prothese gebildet ist aus einem im Inneren des Prothesenschaftes angeordneten und nach außen führenden Absaugkanal (3), von dem aus Absaugöffnungen (5) zur Außenfläche des in den Knochenhohlraum einsetzbaren Prothesenschaftes führen.

2. Endoprothese nach Anspruch 1, dadurch gekennzeichnet, daß die Absaugöffnungen (5) gegenüber den Austrittsöffnungen (4) versetzt sind.

3. Endoprothese nach Anspruch 1 oder 2, dadurch gekennzeichnet, daß sich die Austrittsöffnungen (4) in Austrittsrichtung erweitern.

4. Endoprothese nach einem der voranstehenden Ansprüche, dadurch gekennzeichnet, daß der Innendurchmesser der Austritts- und/oder der Absaugöffnungen (4 beziehungsweise 5) längs der Prothese entsprechend dem von jeder Öffnung (4 beziehungsweise 5) zu versorgenden Teil des Zwischenraumes (14) zwischen Prothese und umgebendem Knochen (10) angepaßt ist.

5. Endoprothese nach einem der voranstehenden Ansprüche, dadurch gekennzeichnet, daß die Austritts- und/oder Absaugöffnungen (4 beziehungsweise 5) im Bereich der Prothesenoberfläche mit einer Schicht eines verformbaren Materials ausgekleidet sind.

6. Endoprothese nach einem der voranstehenden Ansprüche, dadurch gekennzeichnet, daß am distalen Ende der Prothese ein das Eintreten von Knochenzement in die Markraumöffnung des Knochens (10) verhindernder Markraumstopper (6) angeordnet ist.

7. Endoprothese nach Anspruch 6, dadurch gekennzeichnet, daß der Markraumstopper (6) an der Prothese fixiert ist.

8. Endoprothese nach Anspruch 7, dadurch gekennzeichnet, daß der Markraumstopper (6) eine zum distalen Ende der Prothese hin aufspannbare, regenschirmförmig ausgebildete Kappe ist.

9. Endoprothese nach Anspruch 8, dadurch gekennzeichnet, daß die Kappe aufgespannt ist und sich zentrierend an die Innenwand des Markraumes (25) anlegt.

10. Endoprothese nach Anspruch 7, dadurch gekennzeichnet, daß an der Prothese als distaler Markraumstopper (6) ein diese umgebender Ring angeordnet ist.

11. Endoprothese nach Anspruch 10, dadurch gekennzeichnet, daß der Ring ein Schlauch (12) ist, in den ein fließfähiges Medium einführbar ist.

12. Endoprothese nach Anspruch 7, dadurch gekennzeichnet, daß der Markraumstopper (6) als sich an das distale Ende der Prothese anschließender Ballon (16) ausgebildet ist, in den ein fließfähiges Medium einführbar ist.

13. Endoprothese nach einem der Ansprüche 6 bis 12, dadurch gekennzeichnet, daß der Markraumstopper (6) exzentrisch zum Prothesenschaft (1) angeordnet ist.

14. Endoprothese nach einem der Ansprüche 6 bis 13, dadurch gekennzeichnet, daß der Markraumstopper (6) aus einem resorbierbaren Material besteht.

15. Endoprothese nach einem der Ansprüche 6 bis 13, dadurch gekennzeichnet, daß der Markraumstopper (6) aus einem knöchern um- oder einbaubaren Material besteht.

16. Endoprothese nach einem der Ansprüche 6 bis 13, dadurch gekennzeichnet, daß der Markraumstopper (6) aus einem weichen Material besteht.

17. Endoprothese nach einem der Ansprüche 6 bis 16, dadurch gekennzeichnet, daß die Prothese auf der proximalen Seite des distalen Markraumstoppers (6) eine nach innen zurückspringende Ausnehmung (15) zur Aufnahme von Knochenzement aufweist.

18. Endoprothese nach einem der Ansprüche 6 bis 17, dadurch gekennzeichnet, daß der distale Markraumstopper (6) porös ist.

19. Endoprothese nach einem der voranstehenden Ansprüche, dadurch gekennzeichnet, daß an der Prothese als proximaler Markraumstopper (17) ein diese umgebender, gegebenenfalls angeformter Ring (24) angeordnet ist, dessen Außendurchmesser größer ist als der Innendurchmesser des Markraumes (25).

20. Endoprothese nach Anspruch 19, dadurch gekennzeichnet, daß der Ring (24) drehbar beziehungsweise gleitend mit der Prothese verbunden ist.

21. Endoprothese nach einem der Ansprüche 19 oder 20, dadurch gekennzeichnet, daß der Ring (24) abnehmbar ist.

22. Endoprothese nach Anspruch 21, dadurch gekennzeichnet, daß der Ring (24) teilbar ist.

23. Endoprothese nach einem der Ansprüche 19 bis 22, dadurch gekennzeichnet, daß der Ring (24) zumindest an seiner Unterseite aus einem weichen Kunststoffmaterial besteht.

24. Endoprothese nach Anspruch 19, dadurch gekennzeichnet, daß der Ring (24) sich in distaler Richtung soweit verjüngt, daß er mit seinem distalen Ende in die Markraumöffnung einführbar ist.

25. Endoprothese nach Anspruch 24, dadurch gekennzeichnet, daß der Ring (24) als Konus ausgebildet ist.

26. Endoprothese nach Anspruch 24, dadurch gekennzeichnet, daß der Ring (24) gebogene Flanken (26) aufweist.

27. Endoprothese nach Anspruch 26, dadurch gekennzeichnet, daß die Flanken (26) des Ringes (24) längs des Ringumfanges unterschiedlich verlaufen.

28. Endoprothese nach einem der Ansprüche 24 bis 27, dadurch gekennzeichnet, daß die Ebene des Ringes (24) gegenüber der Prothesenschaftachse geneigt ist.

29. Endoprothese nach einem der Ansprüche 19 bis 28, dadurch gekennzeichnet, daß der Ring (24) aus einem im Vergleich zur übrigen Prothese weichen Material besteht.

30. Endoprothese nach Anspruch 29, dadurch gekennzeichnet, daß das Material des Ringes (24) durch Fingerdruck leicht komprimierbar ist.

31. Endoprothese nach einem der Ansprüche 19 bis 30, dadurch gekennzeichnet, daß der Ring (24) aus einem resorbierbaren Material besteht.

## Claims

1. An endoprosthesis which can be cemented in position, having a prosthesis shaft (1) insertable into a bone cavity, having a feed channel (2) for bone cement which is fluid and can harden, having at least one outlet opening (4) which runs from this feed channel (2) to the outer surface of the prosthesis shaft and through which bone cement can be pressed into the gap between prosthesis and surrounding bone, and having a connection in the prosthesis for connecting the gap between prosthesis and surrounding bone to the exterior when the endoprosthesis is inserted in the bone cavity, characterised in that the connection in the prosthesis is formed from a suction channel (3) arranged in the interior of the prosthesis shaft, leading to the exterior and from which suction openings (5) run to the outer surface of the prosthesis shaft insertable into the bone cavity.

2. An endoprosthesis in accordance with Claim 1, characterised in that the suction openings (5) are offset in relation to the outlet openings (4).

3. An endoprosthesis in accordance with Claim 1 or 2, characterised in that the outlet openings (4) widen in the discharge direction.

4. An endoprosthesis in accordance with any one of the preceding Claims, characterised in that the internal diameter of the outlet openings and/or the suction openings (4 and 5 respectively) along the prosthesis is appropriately adapted to that part of the gap (14) between prosthesis and surrounding bone (10) which is to be served by each opening (4 or 5).

5. An endoprosthesis in accordance with any one of the preceding Claims, characterised in that the outlet openings and/or suction openings (4 and 5 respectively) are in the region of the prosthesis surface lined with a coating of a deformable material.

6. An endoprosthesis in accordance with any one of the preceding Claims, characterised in that a bone-marrow-space stop (6) preventing the entry of bone cement into the bone-marrow-space opening of the bone (10) is arranged at the distal end of the prosthesis.

7. An endoprosthesis in accordance with Claim 6, characterised in that the bone-marrow-space stop (6) is fixed to the prosthesis.

8. An endoprosthesis in accordance with Claim 7, characterised in that the bone-marrow-space stop (6) is a cap which is umbrella-shaped and can be opened out in the direction of the distal end of the prosthesis.

9. An endoprosthesis in accordance with Claim 8, characterised in that the cap is opened out and applies itself against the inner wall of the bone marrow space (25) in a centring manner.

10. An endoprosthesis in accordance with Claim 7, characterised in that as a distal stop (6) of the bone marrow space, the prosthesis has a ring arranged on it in a manner so as to surround it.

11. An endoprosthesis in accordance with Claim 10, characterised in that the ring is a hose (12) into which a fluid medium can be introduced.

12. An endoprosthesis in accordance with Claim 7, characterised in that the bone-marrow-space stop (6) is in the form of a balloon (16) which is attached to the distal end of the prosthesis and into which a fluid medium can be introduced.

13. An endoprosthesis in accordance with any one of Claims 6 to 12, characterised in that the bone-marrow-space stop (6) is arranged eccentric to the prosthesis shaft (1).

14. An endoprosthesis in accordance with any one of Claims 6 to 13, characterised in that the bone-marrow-space stop (6) is composed of an absorbable material.

15. An endoprosthesis in accordance with any one of Claims 6 to 13, characterised in that the bone-marrow-space stop (6) is composed of a material which can be placed in an inserted or surrounding manner like a bone.

16. An endoprosthesis in accordance with any one of Claims 6 to 13, characterised in that the bone-marrow-space stop (6) is composed of a soft material.

17. An endoprosthesis in accordance with any one of Claims 6 to 16, characterised in that the prosthesis has - at the proximal side of the distal stop (6) of the bone marrow space - a recess (15) which projects inwards and is for receiving bone cement.

18. An endoprosthesis in accordance with any one of Claims 6 to 17, characterised in that the distal stop (6) of the bone marrow space is porous.

19. An endoprosthesis in accordance with any one of the preceding Claims, characterised in that a ring (24) which surrounds the prosthesis is arranged on the latter as a proximal stop (17) of the bone marrow space and is optionally moulded on the prosthesis, the external diameter of this ring (24) being greater than the internal diameter of the bone marrow space (25).

20. An endoprosthesis in accordance with Claim 19, characterised in that the ring (24) is connected to the prosthesis so as to be rotatable and/or to slide.

21. An endoprosthesis in accordance with either one of Claims 19 or 20, characterised in that the ring (24) is removable.

22. An endoprosthesis in accordance with Claim 21, characterised in that the ring (24) can be taken apart.

23. An endoprosthesis in accordance with any one of Claims 19 to 22, characterised in that at least the underside of the ring (24) is composed of a soft plastics material.

24. An endoprosthesis in accordance with Claim 19, characterised in that the ring (24) tapers in the distal direction to such an extent that its distal end can be inserted into the opening of the bone marrow space.

25. An endoprosthesis in accordance with Claim 24, characterised in that the ring (24) is in the form of a cone.

26. An endoprosthesis in accordance with Claim 24, characterised in that the ring (24) has curved sides (26).

27. An endoprosthesis in accordance with Claim 26, characterised in that the sides (26) of the ring (24) extend in a different manner over the periphery of the ring.

28. An endoprosthesis in accordance with any one of Claims 24 to 27, characterised in that the plane of the ring (24) is inclined in relation to the prosthesis shaft axis.

29. An endoprosthesis in accordance with any one of Claims 19 to 28, characterised in that the ring (24) is composed of a material which is soft compared with the remainder of the prosthesis.

30. An endoprosthesis in accordance with Claim 29, characterised in that the material of the ring (24) is easily compressible by finger pressure.

31. An endoprosthesis in accordance with any one of Claims 19 to 30, characterised in that the ring (24) is composed of an absorbable material.

## Revendications

1. Endoprothèse à implanter à l'aide de ciment, comprenant une tige de prothèse (1) à mettre en place dans une cavité de l'os, un canal d'introduction (2) pour du ciment osseux fluide et durcissable, au moins une ouverture de sortie (4) menant depuis ce canal d'introduction (2) jusqu'à la surface extérieure de la tige de prothèse, ouverture de sortie à travers laquelle du ciment osseux peut être injecté sous pression dans l'espace intermédiaire entre la prothèse et l'os environnant, et une liaison dans la prothèse afin de relier vers l'extérieur l'espace intermédiaire entre la prothèse et l'os environnant lorsque l'endoprothèse est mise en place dans la cavité de l'os,
- caractérisée en ce que la liaison dans la prothèse est formée par un canal d'aspiration (3) agencé à l'intérieur de la tige de prothèse et menant vers l'extérieur, canal d'aspiration depuis lequel des ouvertures d'aspiration (5) mènent jusqu'à la surface extérieure de la tige de prothèse à mettre en place dans la cavité de l'os.

2. Endoprothèse selon la revendication 1, caractérisée en ce que les ouvertures d'aspiration (5) sont décalées par rapport aux ouvertures de sortie (4).

3. Endoprothèse selon l'une ou l'autre des revendications 1 et 2, caractérisée en ce que les ouvertures de sortie (4) s'élargissent dans la direction de sortie.

4. Endoprothèse selon l'une quelconque des revendications précédentes, caractérisée en ce que le diamètre intérieur des ouvertures de sortie et/ou d'aspiration (4, 5) sont adaptés le long de la prothèse en fonction de la partie de l'espace intermédiaire (14) entre la prothèse et l'os environnant (10) qui doit être alimentée depuis chaque ouverture (4, 5).

5. Endoprothèse selon l'une quelconque des revendications précédentes, caractérisée en ce que les ouvertures de sortie et/ou d'aspiration (4, 5) sont revêtues dans la région de la surface de la prothèse d'une couche d'un matériau déformable.

6. Endoprothèse selon l'une quelconque des revendications précédentes, caractérisée en ce qu'il est prévu un bouchon (6) agencé à l'extrémité distale de la prothèse, et empêchant la pénétration de ciment osseux dans l'ouverture du canal médullaire de l'os (10).

7. Endoprothèse selon la revendication 6, caractérisée en ce que le bouchon (6) pour le canal médullaire est fixé à la prothèse.

8. Endoprothèse selon la revendication 7, caractérisée en ce que le bouchon (6) pour le canal médullaire est un capuchon réalisé en forme de parapluie déployable en direction de l'extrémité distale de la prothèse.

9. Endoprothèse selon la revendication 8, caractérisée en ce que le capuchon est déployé et s'applique de façon centrée contre la paroi intérieure du canal médullaire (25).

10. Endoprothèse selon la revendication 7, caractérisée en ce qu'il est prévu en tant que bouchon distal (6) pour le canal médullaire un anneau agencé sur la prothèse et entourant celle-ci.

11. Endoprothèse selon la revendication 10, caractérisée en ce que l'anneau est un tube (12) dans lequel peut être introduit un produit fluide.

12. Endoprothèse selon la revendication 7, caractérisée en ce que le bouchon (6) est réalisé sous la forme d'un ballon (16) raccordé à l'extrémité distale de la prothèse et dans lequel peut être introduit un produit fluide.

13. Endoprothèse selon l'une quelconque des revendications 6 à 12, caractérisée en ce que le bouchon (6) est agencé de façon excentrique par rapport à la tige de prothèse (1).

14. Endoprothèse selon l'une quelconque des revendications 6 à 13, caractérisée en ce que le bouchon (6) est constitué en un matériau résorbable.

15. Endoprothèse selon l'une quelconque des revendications 6 à 13, caractérisée en ce que le bouchon (6) est réalisé en un matériau capable d'être enveloppé ou envahi par les tissus osseux.

16. Endoprothèse selon l'une quelconque des revendications 6 à 13, caractérisée en ce que le bouchon (6) est constitué en un matériau souple.

17. Endoprothèse selon l'une quelconque des revendications 6 à 16, caractérisée en ce que la prothèse comporte sur le côté proximal du bouchon distal (6) un évidement (15) tourné vers l'intérieur afin de recevoir du ciment osseux.

18. Endoprothèse selon l'une quelconque des revendications 6 à 17, caractérisée en ce que le bouchon (6) distal est poreux.

19. Endoprothèse selon l'une quelconque des revendications précédentes, caractérisée en ce qu'il est prévu sur la prothèse en tant que bouchon proximal (17) pour le canal médullaire un anneau (24) qui entoure la prothèse, le cas échéant un anneau conformé, dont le diamètre extérieur est supérieur au diamètre intérieur du canal médullaire (25).

20. Endoprothèse selon la revendication 19, caractérisée en ce que l'anneau (24) est relié en rotation, ou en coulissement, à la prothèse.

21. Endoprothèse selon l'une ou l'autre des revendications 19 et 20, caractérisée en ce que l'anneau (24) est amovible.

22. Endoprothèse selon la revendication 21, caractérisée en ce que l'anneau (24) est subdivisible.

23. Endoprothèse selon l'une quelconque des revendications 19 à 22, caractérisée en ce que l'anneau (24) est constitué au moins à sa face inférieure en une matière plastique souple.

24. Endoprothèse selon la revendication 19, caractérisée en ce que l'anneau (24) se rétrécit en direction distale de telle manière qu'il peut être introduit par son extrémité distale dans l'ouverture du canal médullaire.

25. Endoprothèse selon la revendication 24, caractérisée en ce que l'anneau (24) est réalisé sous la forme d'un cône.

26. Endoprothèse selon la revendication 24, caractérisée en ce que l'anneau (24) présente des flancs (26) incurvés.

27. Endoprothèse selon la revendication 26, caractérisée en ce que les flancs (26) de l'anneau (24) se développent de façon différente le long de la périphérie de l'anneau.

28. Endoprothèse selon l'une quelconque des revendications 24 à 27, caractérisée en ce que le plan de l'anneau (24) est incliné par rapport à l'axe de la tige de prothèse.

29. Endoprothèse selon l'une quelconque des revendications 19 à 28, caractérisée en ce que l'anneau (24) est constitué en un matériau souple par comparaison au reste de la prothèse.

30. Endoprothèse selon la revendication 29, caractérisée en ce que le matériau de l'anneau (24) est susceptible d'être facilement comprimé à l'aide de la pression d'un doigt.

31. Endoprothèse selon l'une quelconque des revendications 19 à 30, caractérisée en ce que l'anneau (24) est constitué en un matériau résorbable.
